# EUROPEAN PATENT APPLICATION

(11) **EP 3 913 355 A1**
(43) Date of publication of application: **24.11.2021**
(21) Application number: 21175539.2
(22) Date of filing: 24.05.2021
(51) Int. Cl.: G01N 21/63, G01N 21/65

(54) **SYSTEM AND METHOD FOR IDENTIFYING A VIRAL COMPOUND**

(30) Priority: 22.05.2020 US 202063028963 P
(71) Applicant: Simmonds Precision Products, Inc., Vergennes, VT 05491 (US)
(72) Inventor: ALLRED, Charles Jeff, Vermont, 05482 (US); ZANONI, Raymond, Iowa, 52498-0505 (US)
(74) Representative: Dehns

(57) **Abstract**

A method of identifying a viral compound, which includes modulating a narrow linewidth laser over a range of frequencies to provide a modulated optical signal that includes a single optical sideband, optically focusing the modulated optical signal with the single optical sideband at a viral sample to excite the viral sample and stimulate an emission of photons therefrom, and detecting amplification of the optical sideband emanating from the viral sample indicating an emission of photons at an acoustic resonance of the viral sample.

## Description

### BACKGROUND

### 1. Field of the Disclosure

The subject invention is directed to acoustic spectroscopy, and more particularly, to a system and method for measuring and identifying the acoustic resonance of a viral compound optically.

### 2. Description of Related Art

The virus which causes COVID-19, SARS-CoV-2 is a viral pathogen that is potentially susceptible to inactivation by way of exciting an acoustic response in the viral structure. Such methods have been proven in the literature with other viruses, such as H3N2. See also U.S. Patent No. 7,497,119 which discloses methods and systems for using resonant acousto-EM energy to disrupt biologic structures such as viruses and bacteria.

In order to determine the resonant frequency or mode that causes the most damage to a virus, the frequency of highest acoustic energy absorption needs to be identified. The subject invention proposes a new and unique system and method for optically identifying that frequency by using acoustic spectroscopy of the virus.

### SUMMARY OF THE DISCLOSURE

The subject invention is directed to a new and useful system and method for optically identifying the acoustic resonance of a virus using acoustic spectroscopy. The method involves the steps of modulating a narrow line width laser (i.e., a laser having a stable single longitudinal mode) over a range of frequencies to provide a modulated optical signal that includes a single optical sideband, optically focusing the modulated optical signal with the single optical sideband at a viral sample to excite the viral sample and stimulate an emission of photons therefrom, and subsequently detecting amplification of the optical sideband emanating from the viral sample indicating an emission of photons at an acoustic resonance of the viral sample.

Preferably, the narrow line width laser is modulated at RF frequencies ranging from 1 GHz to 40 GHz, and it is modulated by way of an optical modulator, which generates the single optical sideband. An example of an optical modulator for generating a single optical sideband is a lithium niobate (LiNbO₃) optical modulator. This can also be accomplished using a dual-parallel Mach-Zehnder modulator, or by way of an appropriate filter.

Amplification of the sideband is detected by measuring the power of the stimulated emission of photons relative to the modulated optical signal. The method further comprises the step of filtering the modulated optical signal to permit detection of the optical sideband alone. It is envisioned that the method may also include the step of destroying the virus at the previously identified acoustic resonance of the viral sample.

The system of the subject invention includes a narrow line width laser (i.e., a laser having a stable single longitudinal mode) for generating an optical signal, an RF signal generator for modulating the optical signal over a range of frequencies, an optical modulator for generating a single optical sideband for the modulated optical signal, means for optically focusing the modulated output signal and the optical sideband at a viral sample to excite the viral sample and stimulate an emission of photons therefrom, and a power meter for detecting amplification of the optical sideband emanating from the viral sample indicating a stimulated emission of photons at an acoustic resonance of the viral sample.

The system further includes a filter upstream from the power meter for filtering the modulated optical signal to permit detection of the optical side band alone. Preferably, the RF signal generator is adapted and configured to modulate the optical signal at RF frequencies ranging from 1 GHz to 40 GHz, and the optical modulator may be a lithium niobate (LiNbO₃) optical modulator. Alternatively, a single optical sideband can be generated using a dual-parallel Mach-Zehnder modulator or an appropriate filter.

These and other features of the system and method of the subject invention will become more readily apparent to those having ordinary skill in the art to which the subject invention appertains from the detailed description of the preferred embodiments taken in conjunction with the following brief description of the drawings.

### BRIEF DESCRIPTION OF THE DRAWINGS

So that those having ordinary skill in the art will readily understand how to make and use the virus identification system and method of the subject invention without undue experimentation, embodiments thereof will be described in detail herein below with reference to the figures wherein:
Fig. 1 is an illustration of a modulated optical signal directed at a virus to determine the acoustic signature of the virus;
Fig. 2 is a schematic view at a quantum level of acoustic phonon generation by an incident photon directed at a viral sample; and
Fig. 3 is a schematic representation of a system for identifying a viral compound which is constructed in accordance with a preferred embodiment of the subject disclosure.

### DETAILED DESCRIPTION OF THE PREFERRED EMBODIMENTS

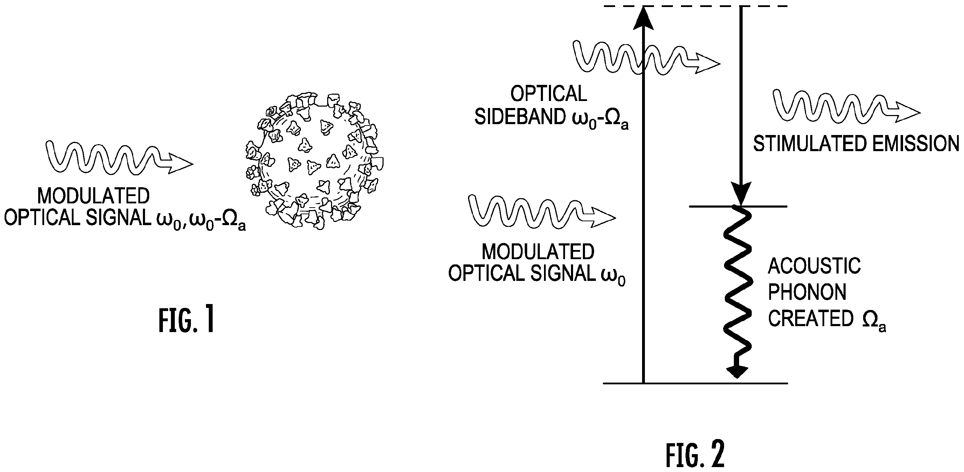

Referring now to the drawings, there is illustrated in Fig. 1 a strategy for performing the viral identification method of the subject disclosure, whereby a radio frequency (RF) signal is directed at a virus to cause electrostriction of the virus, resulting in the creation of acoustic phonons. This strategy is based upon Stimulated Brillouin Scattering, which refers to the interaction of light with the material waves in a medium.

More particularly, there is illustrated in Fig. 1 a modulated optical signal being focused at a virus in such a manner so that acoustic phonons are generated by RF excitation of dipoles within the virus (i.e., molecules in which a concentration of positive electric charge is separated from a concentration of negative charge). This is also known as electrostriction. The RF modulation is tuned to determine the acoustic signature of the virus. Moreover, analyzing the gain of the scattered optical signal enables identification of the acoustic resonance of the virus. By way of example, it is known that the acoustic resonance for the H1N1 virus is ∼8 GHz. That means the V_{sound} in the virus is -800 m/s assuming the virus is ∼100 nm in size.

Referring now to Fig. 2, there is illustrated a quantum level view of the acoustic phonon generation caused by an incident photon and an RF frequency shifted photon, which forms the basis for the method of the subject disclosure. Here, a photon of frequency ω₀ excites the molecule to an excited state, which will emit a photon at a frequency ω₀-Ωₐ and create an acoustic phonon Ωₐ when stimulated by a photon at frequency ω₀-Ωₐ by optically focusing or otherwise directing at a viral sample.

More particularly, the method of the subject disclosure involves modulating a narrow line width laser by way of a signal generator at RF frequencies ranging from 1-40 GHz in such a way that the modulated output has a laser line at ω₀ and a single optical sideband at ω₀-Ω_{g}. The two laser signals ωω₀-Ω_{g} are optically focused onto a specific spot on a viral sample. This can be achieved using one or more lenses, beam expanders, lens couplers or the like.

At such a time, the photons at ω₀ will excite the viral sample to some high energy level, while the single optical sideband at ω₀-Ω_{g} will stimulate the emission of a phonon at ω₀-Ω_{g}, if the virus has an acoustic resonance at Ω_{g}. If the virus does not have that acoustic resonance, the incident RF photon will simply pass on through the sample without stimulating an emission.

However, when there is a stimulated emission at ω₀-Ω_{g}, it can be detected as an amplification of the sideband signal as the RF signal generator is tuned over its range of frequencies. That amplification is then measured as the acoustic resonance or signature of the viral sample. In effect this enables acoustic spectroscopy of the virus optically. Preferably, this is achieved by measuring the power of the sideband signal ω₀-Ω_{g} relative to the power of the output signal Ω_{g} generated by the RF signal generator.

The method further includes the step of filtering the modulated optical signal to permit detection of the optical sideband signal alone. It is envisioned that the method of the subject disclosure may also include the step of destroying the virus at the previously detected and identified acoustic resonance or signature of the viral sample. This can be achieved by direct laser excitation at the acoustic resonance of the virus.

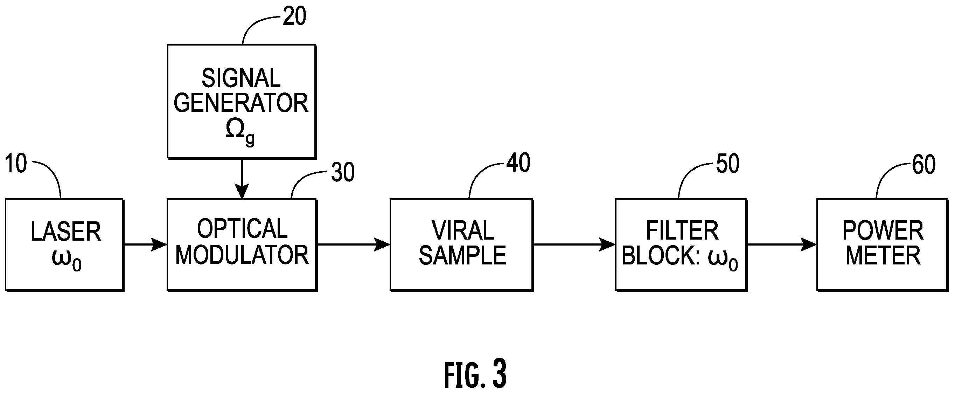

Referring now to Fig. 3, there is illustrated a system for performing the method of the subject disclosure. The system includes a narrow linewidth laser 10 for generating an optical signal. Narrow linewidth lasers are single-frequency lasers with a narrow optical emission spectrum. The system further includes an RF signal generator 20 for modulating the optical signal over a range of frequencies. Preferably, the RF signal generator 20 is adapted and configured to modulate the optical signal at RF frequencies ranging from 1 GHz to 40 GHz.

The system further includes an optical modulator 30 for generating a single optical sideband for the modulated optical signal. An example of such a device is a lithium niobate (LiNbO₃) optical modulator. Other such devices are known in the art and can be employed in this manner. The system also includes optical components for optically focusing the modulated output signal and the optical sideband at a viral sample 40 to excite the viral sample and stimulate an emission of photons therefrom. Such optical components can includes one or more lenses, beam expanders, lens couplers or the like.

The system further includes a power meter 60 for detecting amplification of the optical sideband ω₀-Ω_{g} emanating from the viral sample, which indicates a stimulated emission of photons at an acoustic resonance of the viral sample if the virus has an acoustic resonance at Ω_{g}. The system further includes a filter 50 located upstream from the power meter 60 for filtering the modulated optical signal ω₀ to permit detection of the optical side band ω₀-Ω_{g} alone.

Those skilled in the art will readily appreciate that the system and method of the subject disclosure can be utilized for measuring the acoustic resonances of any viral compound, including the novel coronavirus COVID-19. It should also be readily appreciated that the equipment required to perform spectroscopy in accordance with the subject disclosure should be significantly less expensive than the equipment used to perform traditional Nuclear Magnetic Resonance (NMR) spectroscopy. Additionally, the systems and method of the subject disclosure will directly identify acoustic resonances, rather than requiring their calculation based on the output from traditional NMR spectroscopy. This will allow for the rapid characterization of viral acoustic resonances and a rapid response to new viral pathogens.

While the subject disclosure has been shown and described with reference to preferred embodiments, those skilled in the art will readily appreciate that changes and/or modifications may be made thereto without departing from the scope of the subject disclosure.

## Claims

1. A method of identifying a viral compound, comprising:
a) modulating a narrow line width laser over a range of frequencies to provide a modulated optical signal that includes a single optical sideband;
b) optically focusing the modulated optical signal with the single optical sideband at a viral sample to excite the viral sample and stimulate an emission of photons therefrom; and
c) detecting amplification of the optical sideband emanating from the viral sample indicating an emission of photons at an acoustic resonance of the viral sample.

2. A method according to Claim 1, wherein the narrow line width laser is modulated at RF frequencies ranging from 1 GHz to 40 GHz.

3. A method according to Claim 1 or 2, wherein the narrow line width laser is modulated by way of a lithium niobate (LiNbO₃) optical modulator.

4. A method according to Claim 1, 2 or 3, wherein the narrow line width laser is modulated by way of a dual-parallel Mach-Zehnder modulator.

5. A method according to any preceding claim, wherein amplification of the sideband is detected by measuring the power of the stimulated emission of photons relative to the modulated optical signal.

6. A method according to any preceding claim, further comprising the step of filtering the modulated optical signal to permit detection of the optical sideband alone.

7. A method according to any preceding claim, further comprising the step of destroying the virus at the acoustic resonance of the viral sample.

8. A system for identifying a viral compound, comprising:
a) a laser for generating an optical signal;
b) an RF signal generator for modulating the optical signal over a range of frequencies; and
c) an optical modulator for generating a single optical sideband for the modulated optical signal.

9. A system as recited in Claim 8, further comprising means for optically focusing the modulated output signal and the optical sideband at a viral sample to excite the viral sample and stimulate an emission of photons therefrom.

10. A system as recited in Claim 9, further comprising a power meter for detecting amplification of the optical sideband emanating from the viral sample indicating a stimulated emission of photons at an acoustic resonance of the viral sample.

11. A system as recited in Claim 10, further comprising a filter upstream from the power meter for filtering the modulated optical signal to permit detection of the optical side band alone.

12. A system as recited in any one of claims 8 to 11, wherein the laser is a narrow line width laser.

13. A system as recited in any one of claims 8 to 12, wherein the RF signal generator is adapted and configured to modulate the optical signal at RF frequencies ranging from 1 GHz to 40 GHz.

14. A system as recited in any one of claims 8 to 13, wherein the optical modulator is a lithium niobate (LiNbO₃) optical modulator.

15. A method according to any one of claims 8 to 14, wherein the optical modulator is a dual-parallel Mach-Zehnder modulator.
